# EUROPEAN PATENT APPLICATION

(11) **EP 3 925 450 A1**
(43) Date of publication of application: **22.12.2021**
(21) Application number: 20754993.2
(22) Date of filing: 07.02.2020
(51) Int. Cl.: A23C 9/158, A61P 1/00, A61P 37/04, A23L 33/15, A23L 2/52, A23L 2/38, A61K 31/706

(54) **FOOD OR BEVERAGE FOR BABIES AND INFANTS, METHOD FOR IMPROVING INTESTINAL ENVIRONMENT OF BABY OR INFANT, AND METHOD FOR ENHANCING IMMUNITY OF BABY OR INFANT**

(30) Priority: 12.02.2019 JP 2019022222
(71) Applicant: Tanaka, Megumi, Chigasaki-shi, Kanagawa 253-0054 (JP); Tanaka, Tsunemaru, Chigasaki-shi, Kanagawa 253-0054 (JP)
(72) Inventor: Tanaka, Megumi, Chigasaki-shi, Kanagawa 253-0054 (JP); Tanaka, Tsunemaru, Chigasaki-shi, Kanagawa 253-0054 (JP)
(74) Representative: IPAZ
(86) International application number: PCT/JP2020/004947
(87) International publication number: WO 2020/166527

(57) **Abstract**

PROBLEM: To provide a food, a drink, or the like for infant that allows improving an intestinal environment and enhancing an immune strength of an infant.

SOLUTION: A food or drink for infant containing an effective dose of nicotinamide mononucleotide.

## Description

### TECHNICAL FIELD

The present invention relates to a food or drink for infant, a method for improving an intestinal environment of infant, and a method for enhancing an immune strength of infant.

### BACKGROUND ART

A powdered milk for childcare (hereinafter simply referred to as a "powdered milk" in some cases) is a dietary supplement used as an alternative to a breast milk, for example, when the breast milk is not produced or slightly insufficient with some reason, when a mother has acquired an infection or uses a medicinal product, and when breastfeeding is painful or troublesome. In considering that the breast milk is a main nutrient source for a baby necessary for growth and development of the baby, it is understood that the powdered milk is a food product that has the most important role for the healthy development of the baby. Therefore, the powdered milk is originally required to sufficiently have nutrition necessary for the growth of the baby similarly to the breast milk, and then, powdered milk manufacturers have conducted studies and developments with a target to make a composition of a powdered milk similar to a composition of the breast milk as much as possible, thus trying to meet the request. Consequently, the powdered milks currently produced in our country (Japan) contain considerably improved nutrient ingredients, and various powdered milks with high-qualities are productized from the powdered milk manufacturers. Then, along with the gradual improvement of the qualities of the powdered milk products, the demand for the powdered milk have increased worldwide, and the global market of the powdered milk is expected to expand with a high average annual growth rate in the future. Note that a "formulated milk powder" is defined as "one produced by processing a raw milk, a cow's milk, or a special milk, or a food product manufactured with those as a raw material, or using them as a major raw material, and adding a nutrient necessary for infant thereto and powdering it" in Ministerial Ordinance on Ingredient Standards for Milk and Milk Products in our country (Japan).

Now, the breast milk not only functions as a nutrient source necessary for the growth and the development of the baby, but also variously functions in immunity, mental development, and the like, and these functions are expressed by various kinds of ingredients contained in the breast milk. Specifically describing this respect, the breast milk contains energy sources and nutrients, such as sugar, protein, and lipid, necessary for healthy development of the baby, and contains various infection prevention factors, such as immunoglobulin, a complement, lactoferrin, and lysozyme, for protecting the baby from diarrhea and other infections. These infection prevention factors effectively act to protect the baby from acquiring the infection.

In contrast, while the powdered milk used as an alternative to the breast milk contains the improved nutrient ingredients, it is not necessarily said to be improved in physiologically active ingredients involved in infection prevention and the like. For example, since an antibody generated in a mother body is contained only in the breast milk, completely substituting for the breast milk is impossible. Possibly for this reason, it is pointed out in some people that the baby fed by the powdered milk easily gets a disease because the immunity from the mother is not received. The infancy, especially the neonatal period is said to be in a kind of immunodeficiency state, and because of this, it is said to be easy to acquire the infection.

To deal with such problems, a powdered milk with the reinforced infection prevention function has been produced. This powdered milk contains ingredients known as infection prevention factors contained in the breast milk, such as sialic acid, lactoferrin, and ganglioside, and an infection prevention function as a biological defense mechanism is reinforced. The sialic acid contained in the breast milk is contained in a sugar chain constituting oligosaccharide, glycolipid, and glycoprotein. The lactoferrin as a glycoprotein contains sialic acid at the end of its sugar chain. The ganglioside as a glycolipid has a structure in which glucose, galactose, N-acetylgalactosamine, N-acetylglucosamine, and sialic acid are bound to ceramide containing fatty acid and sphingosine. An infection with pathogen, such as viruses and bacteria, generally begins from binding to the sugar chain containing sialic acid existing in epithelial cells of a mucous membrane, and it is said that a toxin produced by a pathogen, such as pathogenic Escherichia coli and Vibrio cholerae, causes diarrhea and inflammation by binding to the sialic acid-containing sugar chain of gastrointestinal tract epithelial cells. In considering such an infection mechanism, it is considered that the sialic acid-containing component in the breast milk is bound to the pathogens before the pathogens attach to the epithelial cells in an oral cavity, a pharynx, and gastrointestinal tracts of a baby, and inactivate the pathogens, thereby avoiding the infection from outside.

Conventionally, as a specific example of the powdered milk provided with the infection prevention function, for example, Patent Document 1 discloses a childcare powdered milk provided with an infection prevention function. The powdered milk contains sialic acid-binding peptide, which is obtained by performing a protease treatment to sialic acid-binding protein derived from cow's milk, as an infection protective ingredient.

Patent Document 2 discloses a powdered milk created based on the knowledge that ganglioside of human milk and cow's milk have an activity to block attachment of pathogenic microorganisms and viruses to intestinal mucosal cells, that is, a powdered milk to which a ganglioside expressed by a general formula (NANA) n-Gal-Glc-ceramide (note that, in the formula, NANA is N-Acetylneuraminic acid, Gal is galactose, Glc is glucose, and ***n*** is an integer of 1 or 2) is added by 0.5 mg weight% or more.

Meanwhile, while the intestine has an immune function to remove dangerous pathogenic germs, intestinal bacteria allowed by the immunity exist. The intestinal bacteria acting in the intestine are roughly divided into beneficial bacteria, bad bacteria, and opportunistic bacteria. The beneficial bacteria include bifidobacteria, lactic acid bacteria, enterococci, and the like. The bad bacteria include Clostridium perfringens, Staphylococcus aureus, colon bacillus (toxic strain), and the like. The opportunistic bacteria include bacteroides, colon bacillus (avirulent strain), streptococci, and the like. The beneficial bacteria acidify inside the intestinal tract, and are involved in maintaining the health, such as enhancing the immune strength of the gastrointestinal tract, producing vitamin, and reducing cholesterol. Meanwhile, it is said that the bad bacteria decrease the peristaltic action, alkalinize inside the intestinal tract, produce harmful substances and carcinogen, such as toxic ammonia, amine, and hydrogen sulfide, and have harmful actions on the body, such as reducing the immune strength. While the opportunistic bacteria usually have almost no influence, the opportunistic bacteria have a property of participating in the dominant one of the beneficial bacteria or the bad bacteria. While these bacteria coexist in balance with one another, the composition of bacteria varies depending on aging, stress, infection, eating habit, use of antibiotic, and the like.

Now, the gastrointestinal tract of the baby fed by the breast milk internally has a bacterial flora in which bifidobacteria as beneficial bacteria are dominant, suppresses propagation of pathogenic germs, and significantly contributes to the healthy development of the baby. However, it is reported that the baby fed by the powdered milk has far less bifidobacteria in the intestine than the baby fed by the breast milk, and the number of the colon bacillus referred to as bad bacteria is often detected by ten times or more. One of the reasons of this deterioration of balance of the intestinal flora includes that the breast milk contains a bifidus growth factor that promotes the growth of bifidobacteria while the powdered milk does not contain such a factor. Therefore, to improve the intestinal environment of the baby, galacto-oligosaccharide (oligosaccharide of disaccharide to hexasaccharide obtained by causing β-galactosidase to act to lactose), lactulose, raffinose, and the like known as the bifidus growth factors are combined in the powdered milk.

For example, Patent Document 3 discloses a formulation for the purpose of promoting the growth of bifidobacteria occupying the intestinal flora. The formulation contains one or two or more oligosaccharides selected from the group consisting of lactulose, fructo-oligosaccharide, and galacto-oligosaccharide, raffinose, and ingredients for powdered milk.

While it is known that the infants easily acquire (are highly susceptive to) intestinal tract pathogen infections, conventionally, it has been considered that this is caused by immaturity of the immune system. However, recently, it has been gradually revealed that the intestinal flora plays an important role in the development of the immune system and the infection prevention against the intestinal tract pathogens. It has been known that the intestinal flora changes in three years after birth, and matures to the adult intestinal flora. Then, while the infants are highly susceptive to the intestinal tract pathogen infections, it has been proved that the high susceptivity to the intestinal tract pathogen infections of the infants is caused by the immaturity of the intestinal flora. Accordingly, especially to the infants, it is desired to combine the ingredient that can compensate the immaturity of the intestinal flora in a food or drink for infant including the powdered milk.

Patent Document 1: JP-2514375
Patent Document 2: JP-B-6-85684
Patent Document 3: JP-A-10-175867

### DISCLOSURE OF THE INVENTION

### PROBLEMS TO BE SOLVED BY THE INVENTION

As described above, since the baby fed by the conventional powdered milk has a disturbed balance between bifidobacteria and the like as the beneficial bacteria and colon bacillus and the like as the bad bacteria in the intestinal flora compared with the baby fed by the breast milk, the baby fed by the conventional powdered milk tends to more easily acquire an infection in some cases because of the deteriorated intestinal environment or the insufficient immune strength. Therefore, it has been waited to develop a food or drink for infant with reinforced intestinal environment improving ability and immunostimulatory ability (infection prevention function).

Accordingly, it is an object of the present invention to provide a food or drink for infant that allows improving an intestinal environment and enhancing an immune strength of the infant, a method for improving the intestinal environment of the infant, and a method for enhancing the immune strength of the infant.

### SOLUTIONS TO THE PROBLEMS

The inventor seriously studied in order to solve the above-described problems and found that a nicotinamide mononucleotide as an intermediate metabolite involved in biosynthesis of a coenzyme NAD (nicotinamide adenine dinucleotide) can provide an excellent effect of improving the intestinal environment and enhancing the immune strength (infection prevention effect) of the baby. Thus, the present invention was completed.

The present invention is as follows.
[1] A food or drink for infant that contains an effective dose of nicotinamide mononucleotide.
[2] The food or drink for infant according to [1] used for improving an intestinal environment.
[3] The food or drink for infant according to [1] or [2] used for enhancing an immune strength.
[4] In the food or drink for infant according to any of [1] to [3], the infant is a toddler.
[5] In the food or drink for infant according to any of [1] to [4], the nicotinamide mononucleotide is contained with an amount provided in a range of 0.2 to 220 mg per day for one baby in terms of nicotinamide mononucleotide amount.
[6] In the food or drink for infant according to any of [1] to [5], the food or drink for infant is a powdered milk for childcare.
[7] A method for improving an intestinal environment of a baby that includes a step of causing a baby whose intestinal environment is deteriorated to ingest the food or drink for infant according to any of [1] to [6].
[8] A method for enhancing an immune strength of a baby that includes a step of causing a baby whose immune strength is low to ingest the food or drink for infant according to any of [1] to [6].
[9] In the method according to [7] or [8], an intake of the food or drink for infant is an amount in a range of 0.2 to 220 mg per day for one infant in terms of nicotinamide mononucleotide amount contained in the food or drink.

### EFFECTS OF THE INVENTION

The present invention provides the food or drink for infant that allows improving the intestinal environment and enhancing the immune strength of the infant and is excellent in safety.

### BRIEF DESCRIPTION OF THE DRAWINGS

FIG. 1 is an explanatory drawing illustrating a metabolic pathway involved in niacin (generic term of nicotinamide and nicotinic acid).
FIG. 2 is a graph illustrating a result of Example 1.
FIG. 3 is a graph illustrating a result of Example 2.
FIG. 4 is a graph illustrating a result of Example 3

### DESCRIPTION OF PREFERRED EMBODIMENTS

A food or drink for infant according to the present invention is a food or drink for infant that contains nicotinamide mononucleotide as a physiologically active ingredient in addition to ordinary nutrient ingredients, thereby improving an intestinal environment and enhancing an immune strength of an infant, thus reinforcing a physiologically activity to protect the infant against the infection caused by pathogenic germs and viruses. In the present invention, the "improvement of the intestinal environment of the infant" means that a proportion of the number of bacteria of bifidobacteria and lactic acid bacteria as beneficial bacteria in the intestinal bacteria increases, thus forming an intestinal flora in which the beneficial bacteria are dominant. Here, the "intestinal flora" is defined as a wide variety of microbial communities living in the intestine. When the proportion of the beneficial bacteria increases, the influence of the bad bacteria relatively decreases, a satisfactory balance of the intestinal flora is kept by a state where the beneficial bacteria are dominant, thus providing an excellent intestinal environment. Then, the bad bacteria cannot actively act to harm the health of infant, resulting in improving the health condition of the infant. It is lymph corpuscles as a kind of leukocytes that play a role in an immune system to protect the body from an external enemy, the lymph corpuscles exist the most in the small bowel and the large bowel, and the immune system is referred to as an intestinal tract immunity. When the satisfactory balance of the intestinal flora is broken, the act of the intestinal tract immunity is reduced, thus easily causing an infection, such as stomatitis and cold. That is, keeping the satisfactory balance of the intestinal flora allows normally acting of the intestinal tract immunity, thereby providing the high immune strength.

Generally, in the forming process of the intestinal flora of the baby in one month after birth, first, an intestinal flora composition in which any of staphylococcus, colon bacillus, and bifidobacterium is the most dominant bacterium is formed, and subsequently, the composition transitions to an intestinal flora composition in which bifidobacterium is dominant. While the individual difference is recognized in the transition timing, the intestinal flora in which bifidobacterium is the most dominant bacterium is formed in the intestinal tract of most babies. For example, bifidobacterium gradually increases after the elapse of about three days after birth, reaches 10 to 100 billion per gram of feces on the fourth to seventh day, and occupies 95% or more of intestinal bacteria in the breast-fed baby, while the numbers of colon bacilli and enterococci decrease to about one hundredth of bifidobacteria.

Meanwhile, in the example of a baby fed with the conventional common powdered milk, the coliform group is still the most dominant on the sixth day after birth, and while bifidobacteria become dominant in one month after birth, the proportion is low in some cases compared with the intestinal flora of the breast-fed baby in which bifidobacteria are overwhelmingly dominant. A recent study reveals that the intestinal flora composition in infancy significantly affects the physiological function of the individual after growth.

In the present invention, while the detailed reason why containing the nicotinamide mononucleotide as the physiologically active ingredient providing the improved intestinal environment and the enhanced immune strength provides the effects of the present invention is currently examined, one conceivable reason is as follows. That is, since nicotinamide mononucleotide acts as a substance that promotes the growth of bifidobacteria and lactic acid bacteria as the beneficial bacteria, and has an action to selectively increase bifidobacteria and lactic acid bacteria, consequently, the intestinal flora composition becomes a flora in which the beneficial bacteria are dominant. Then, with the increase of the beneficial bacteria, organic matters such as lactic acid and acetic acid are generated as their metabolites, the increase of the generation of lactic acid and acetic acid acidifies inside the intestine, and the growth of the bad bacteria, such as colon bacilli and Clostridium perfringens, that prefer the alkaline environment is suppressed. Consequently, the healthy intestinal environment in which the beneficial bacteria are dominant can be obtained, thus improving the intestinal environment. When the proportion of the beneficial bacteria is increased, the influence of the bad bacteria relatively decreases and the beneficial bacteria become dominant, thereby keeping the satisfactory balance of the intestinal flora. Then, the bad bacteria cannot actively act to harm the toddler's health, resulting in enhancing the immune strength of the toddler. The increased lactic acid bacteria and its cell component stimulate an innate immune receptor of the intestinal tract immunity on the epithelial cells of the small bowel as a stage of the intestinal tract immunity, and this stimulation generates a large number of cytokines that suppress the growth of the pathogenic germs and antimicrobial substances are secreted. Additionally, the infection with pathogenic germs and viruses entered from outside is avoided at an early stage, thereby providing a biological defense action and an immunostimulatory action. This is also considered as the reason. It is also considered that nicotinamide mononucleotide has an action of growing lymph corpuscles as one of immune cells and an action of promoting immunoglobulin production. Since the small bowel is an organ in which immune cells and peripheral blood vessels are concentrated, when the intestinal environment is right, the biological defense function and a disease prevention function are enhanced, thus providing a positive influence on the health. The lactic acid bacteria have a function to make the intestinal environment right such that digestion and absorption of nutrients as a role of the small bowel is appropriately performed.

The nicotinamide mononucleotide (chemical formula: C₁₁H₁₅N₂O₈P) is a compound produced in bodies of many organisms including human, and expressed with a structural formula [Chem. 1] below. The nicotinamide mononucleotide is generally referred to as NMN, and known as an intermediate metabolite involved in a biosynthesis of coenzyme NAD⁺.

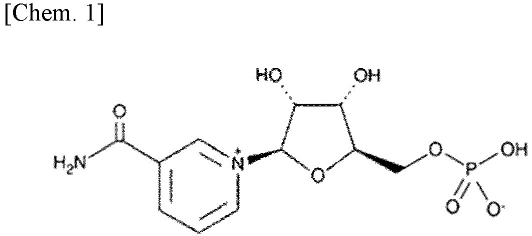

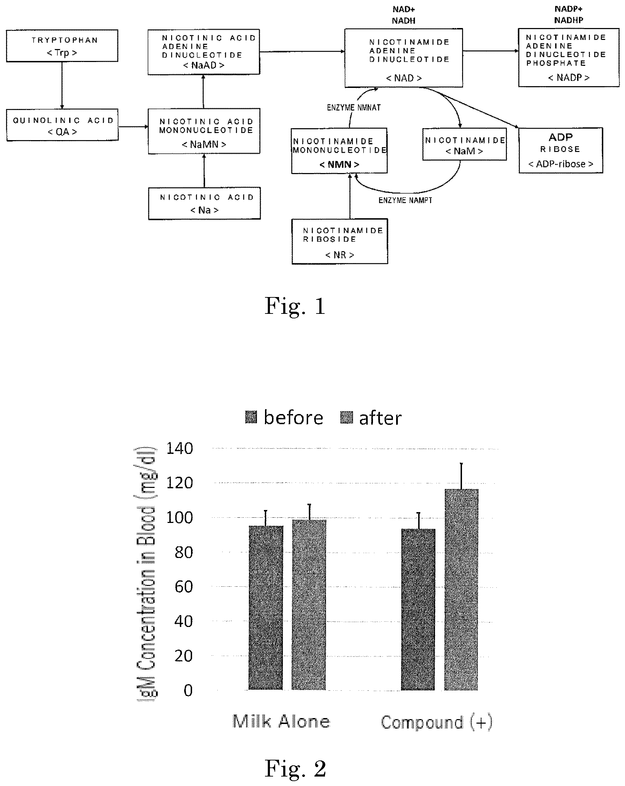

The nicotinamide mononucleotide combined in the food or drink for infant according to the present invention is produced in an NAD metabolic pathway by liver tissues, that is, a pathway involved in a synthesis of a nicotinamide adenine dinucleotide (NAD) from a quinolinic acid through a kynurenine pathway, in vivo. This will be specifically described with reference to FIG. 1. FIG. 1 is an explanatory drawing illustrating a metabolic pathway involved in niacin (generic term of a nicotinamide and a nicotinic acid) known as vitamin B₃. The nicotinic acid ingested through a meal is absorbed by the liver to be converted into nicotinamide, and the nicotinamide is supplied to the whole body via a blood flow. The cells each absorb the nicotinamide from the blood, and convert it into the NAD and an NADP to use them. The nicotinamide is biosynthesized also from a tryptophan.

As illustrated in FIG. 1, in vivo, when the tryptophan is a starting material, the tryptophan is converted into the quinolinic acid (QA) through the kynurenine pathway as a tryptophan metabolic pathway, and further converted into a nicotinic acid mononucleotide (NaMN). Meanwhile, when the nicotinic acid (Na) is the starting material, the nicotinic acid is directly converted into the NaMN Afterwards, the NaMN is interconverted into the NAD, a nicotinamide (NaM), and the nicotinamide mononucleotide in a NAD cycle through a nicotinic acid adenine dinucleotide (NaAD). The nicotinamide (NaM) is converted into the nicotinamide mononucleotide by a nicotinamide phosphoribosyltransferase (NAMPT), subsequently, the nicotinamide mononucleotide is converted by a nicotinamide mononucleotide adenyltransferase (NMNAT) to generate the NAD. Note that, the nicotinamide mononucleotide is produced also from a nicotinamide riboside (NR) as an NAD intermediate metabolite.

The nicotinamide mononucleotide includes two types of an α-form and a β-form as optical isomers, and the β-form is used in the present invention. The nicotinamide mononucleotide is obtained by, for example, synthesizing a nicotinamide riboside from the nicotinamide and a ribose (see Bioorg. Med. Chem. Lett., 12, 1135-1137 (2002)), and subsequently, phosphorylating a 5-hydroxyl group of the ribose part (see Chem. Comm., 1999, 729-730). Specifically, for example, first, a reaction solution is prepared by dissolving the nicotinamide and an L-ribose tetraacetate in anhydrous acetonitrile, adding a trimethylsilyl trifluorosulfonic acid by an excessive amount under a nitrogen stream and then stirring at room temperature, and adding methanol to stop the reaction. The above-described reaction solution is poured into a column filled with activated carbon, cleaned with a distilled water, and then eluted with methanol and its product is collected. Next, for a phosphorylation reaction of the 5-hydroxyl group of the L-ribose part of this product, a reaction solution is prepared by dissolving the above-described product in a trimethoxy phosphoric acid, dropping a phosphorus oxychloride below freezing and stirring under the nitrogen stream, adding a sodium hydroxide aqueous solution to neutralize, thus stopping the reaction. A cold acetonitrile-ether solution is added to the above-described reaction solution. Afterwards, a lower layer (water phase) is passed through an anion-exchange resin to collect a reactant, and further purifies the reactant with a cation-exchange resin, thus the high-purity nicotinamide mononucleotide can be collected. The nicotinamide mononucleotide is commercially available from Oriental Yeast Co., ltd., and Bontac Bio-engineering (Shenzhen) Co., Ltd., and those commercial products can be purchased for use.

The nicotinamide mononucleotide is a purified product that contains a few impurities, especially, preferably its purity is 90% or more, and further preferably its purity is 95% or more. When the purity is less than 90%, a bad smell possibly occurs, or the effect of the nicotinamide mononucleotide is possibly reduced to fail to sufficiently provide the effect of the present invention.

While the purity of the nicotinamide mononucleotide is preferably 90% or more as described above, the purity (mass ratio) is defined as a value obtained by subtracting the impurities other than the nicotinamide mononucleotide from 100% in anhydrous terms. Accordingly, the purity of the nicotinamide mononucleotide can be obtained with a formula: nicotinamide mononucleotide purity (%) = 100 - impurities other than nicotinamide mononucleotide (%). Here, these impurities include, as illustrated in FIG. 1, metabolites excluding the nicotinamide mononucleotide involved in the NAD metabolic pathway, especially, the nicotinamide and the nicotinamide adenine dinucleotide. When the nicotinamide mononucleotide used in the present invention contains a foreign element such as the above-described metabolite involved in the NAD metabolic pathway, for example, the absorption of the nicotinamide mononucleotide into living cells possibly reduces, resulting in the reduction of the effect of the present invention. A quantitative determination of the above-described impurities involved in the NAD metabolic pathway is performed with an absolute calibration curve method using a standard sample where a test solution of dried nicotinamide mononucleotide powder is poured into an HPLC apparatus, and a peak area of an obtained chromatograph is obtained (vertical axis: peak area, horizontal axis: concentration). Since the use of the peak height ensures the quantitative determination with high accuracy in a case of a trace substance, the apparatus to be used is appropriately chosen according to the characteristics of the apparatus. Separated substances are identified based on retention times.

The food or drink for infant according to the present invention is a food or drink for infant that contains nicotinamide mononucleotide as a physiologically active ingredient by an amount enough to provide the effect of the present invention, that is, an "effective dose" specified in claim 1 when each food or drink is taken by an ordinary intake. The food or drink for infant according to the present invention is a food or drink for infant that has functions based on an action to improve an intestinal flora and an action to promote an immune function of an infant together by combining nicotinamide mononucleotide as a physiologically active ingredient having the above-described actions in a food or drink for infant ingested in daily life. The "food or drink for infant" is a concept widely including foods or drinks for babies and toddlers, the type of food is not specifically limited, and the form may be any of a solid state, a semisolid state, and a liquid state. Specifically, a powdered milk for childcare, a liquid milk, a baby food, a baby drink, a baby dessert, a baby rice-seasoning, confectionery for infant (bread, gum, candy, tablet, cookie, gummi candy, biscuit, cake, chocolate, Japanese confectionery, jelly, and the like), a dairy product (yogurt, cheese, milk, butter, and the like), ice cream, cream, jam, a nutritious food, a medication support jelly, and the like are included. Among these foods or drinks for infant, from the aspect of the high necessity and usability of the effect of the present invention, the powdered milk for childcare is especially preferred. This is because the intestinal environment of the toddler is unstable and the immune system is immature, and the powdered milk for childcare is the most important food or drink that contains the nutrient source for toddlers. The powdered milk for childcare includes any powdered milk used for artificial feeding of baby including a special milk, a lactose-free milk, and a milk for milk allergy in addition to a formulated milk powder for babies, a follow-up milk, and a milk for low-birth-weight baby. The liquid milk is a liquid state milk in which nutrient ingredients are adjusted so as to allow the baby to take the liquid milk instead of the breast milk. The baby food is a food produced for the purpose of nutrition support and sequentially adapting the baby to general foods in association with the growth of the baby. The baby food includes a canned food, a bottled food, and a packed food of meat, grain, vegetable, fruit, mineral, and the like, which are processed to pastes, and one eaten in a form of a paste by adding hot water or water to a powder, a granule, a flake, and a solid of a dried material of them. The baby drink is a food produced for the purpose of hydration, nutrition support, and weanling support of the baby. Here, the "infant" is a concept including the baby and the toddler. The "baby" means a child until about one year after birth who takes the nutrition from the breast milk and the milk and is in a period before starting walking. The "toddler" means a child of about one to six years after birth in a period of walking with his/her own feet and taking the nutrition from a meal other than the breast milk or the milk.

In the food or drink for infant according to the present invention, the combined amount of nicotinamide mononucleotide only needs to be an effective dose, that is, an amount with which nicotinamide mononucleotide as the physiologically active ingredient can provide the effect of the present invention when each food or drink is ingested by the ordinary intake, and may be appropriately determined for each food or drink in consideration of the influence on the taste of the food or drink (nicotinamide mononucleotide is acid in taste), the intake, the cost, and the like.

In the food or drink for infant, the ingredient composition other than nicotinamide mononucleotide is not specifically limited, and known ingredients of each food or drink are appropriately combined depending on the type and the like of each food or drink. For example, in the case of the powdered milk for childcare, the ingredient composition is generally a heat amount of 60 to 70 kcal/100 ml, protein of 1.8 to 3.0 g/100 kcal, carbohydrate of 9.0 to 14.0 g/100 kcal, and lipid of 4.4 to 6.0 g/100 kcal, and as the other ingredients, various kinds of vitamins, minerals, and various kinds of oligosaccharides having prebiotic actions are combined, and lactoferrin, ganglioside, sialic acid, and the like are combined as the infection prevention factors.

A period immediately after birth to a weanling period is an important period for establishing intestinal bacteria, and the intestinal bacteria inhabited during this period are subjected to various processes to form the intestinal flora of each individual. Taking into account the fact that the intestinal flora composition in infancy significantly affects the physiological function of the individual after growth, and the intestinal environment of the toddler is unstable and the immune system is immature as described above, it can be said that the food or drink for infant according to the present invention is highly beneficial especially to the baby.

According to a preferred aspect of the present invention, a food or drink for infant used for improving an intestinal environment is provided. A food or drink for infant used for enhancing an immune strength and protection against an infection is provided.

When the food or drink for infant according to the present invention is ingested, the intake can be appropriately determined in considering the age (age in month), sex, intestinal flora state, form and type of food, ingesting time, and the like of the infant. However, to effectively obtain the effect of the present invention, the intake is ordinarily an amount of 0.2 to 220 mg per day for one infant in terms of nicotinamide mononucleotide amount, and especially 1 to 220 mg is preferred. In the case of less than 0.2 mg, the effect of the present invention is possibly not sufficiently obtained, and meanwhile, even in the case of increasing to more than 220 mg, the obtained effect is not especially changed, and an economic disadvantage is provided. Accordingly, the food or drink for infant according to the present invention preferably contains nicotinamide mononucleotide by the amount provided in the range of 0.2 to 220 mg, especially 1 to 220 mg per day for one infant in terms of nicotinamide mononucleotide amount. In the case of ingesting by the baby, the intake is decreased compared with the toddler, and is ordinarily an amount of 0.2 to 120 mg per day for one baby in terms of nicotinamide mononucleotide amount, and especially 1 to 120 mg is preferred. The intake specified here can be ingested with an ingesting unit divided into one to several times per day.

The ingesting period of the food or drink for infant according to the present invention is not specifically limited, and it may be appropriately determined while observing the effect. However, in considering that the condition of the intestinal flora is not fixed and can constantly change, not the temporary ingesting for a short period, but the continuous ingesting for a long period is preferred. When the ingesting of the food or drink for infant according to the present invention is halted, the bad bacteria increase to possibly break the balance of the intestinal flora in an undesirable direction. The food or drink for infant according to the present invention has no problem in safety even in the case of the continuous ingesting.

The food or drink for infant according to the present invention is one in which nicotinamide mononucleotide is added to a food or drink for infant, and is manufactured in accordance with an ordinary manufacturing method of each food or drink for infant. The nicotinamide mononucleotide is distributed in the market and commercially available (Oriental Yeast Co., ltd., Bontac Bio-engineering (Shenzhen) Co., Ltd.). For the nicotinamide mononucleotide, recently, the quality control system and the mass production system of nicotinamide mononucleotide are established.

The present invention is a method for improving an intestinal environment of an infant that includes a step of causing an infant whose intestinal environment is deteriorated to ingest the food or drink for infant according to the present invention. In this method, while the amount of the food or drink for infant to be ingested by the infant may be appropriately determined, the amount of the food or drink for infant is preferably the above-described amount per day for one baby in terms of nicotinamide mononucleotide amount. In this method, another step for improving another intestinal environment, for example, a step of causing galacto-oligosaccharide, lactulose, raffinose, and the like known as bifidus growth factors to be ingested can be added, thereby allowing an expectation of the further enhanced effect of the present invention. The period for ingesting the food or drink for infant according to the present invention is as described above.

The present invention is a method for enhancing an immune strength of an infant that includes a step of causing an infant whose immune strength is low to ingest the food or drink for infant according to the present invention. In this method, the amount of the food or drink for infant to be ingested by the infant is similar to the above-described one. In this method, another step for enhancing another immune strength (infection prevention power), for example, a step of causing an ingredient known as the infection prevention factor, such as sialic acid, lactoferrin, and ganglioside, to be ingested can be added, thereby allowing an expectation of the further enhanced effect of the present invention. The period for ingesting the food or drink for infant according to the present invention is as described above.

### [Examples]

Next, while the present invention will be further specifically described with examples, the present invention is not limited to these examples.

### Example 1. Evaluation of Immunoglobulin M (IgM) Production promoting effect

For IgM as an antibody produced from B-cells at first when being infected with bacteria or viruses, to confirm the change of the IgM concentration in blood by ingesting the food or drink for infant containing nicotinamide mononucleotide, a test was conducted on boys and girls aged 1.8 to 6.5 years. The 23 toddlers were divided into two groups, one group (11 toddlers) was fed with a powdered milk alone and the other group (12 toddlers) was fed with a powdered milk (compound (+)) to which nicotinamide mononucleotide was added. The powdered milks were given every day in the morning once a day continuously for about 30 days. The IgM concentration in blood (mg/dl) was measured for each group before feeding with the powdered milk and after feeding with the powdered milk for about 30 days, and the average value was obtained (excluding a subject whose IgM concentration in blood before feeding with the powdered milk was less than 120 mg/dl). In the group fed with the powdered milk to which nicotinamide mononucleotide was added, the powdered milk containing nicotinamide mononucleotide of 100 mg per day was given. FIG. 2 illustrates the result. As illustrated in FIG. 2, in the group fed with the powdered milk to which nicotinamide mononucleotide was added, the IgM concentration more increased from before feeding with the milk to after feeding with the milk than in the group fed with the powdered milk alone. From this result, it was found that the food or drink for infant of the present invention was effective for increasing the IgM concentration in blood.

### Example 2. Evaluation of Immunoglobulin A (IgA) Production promoting effect

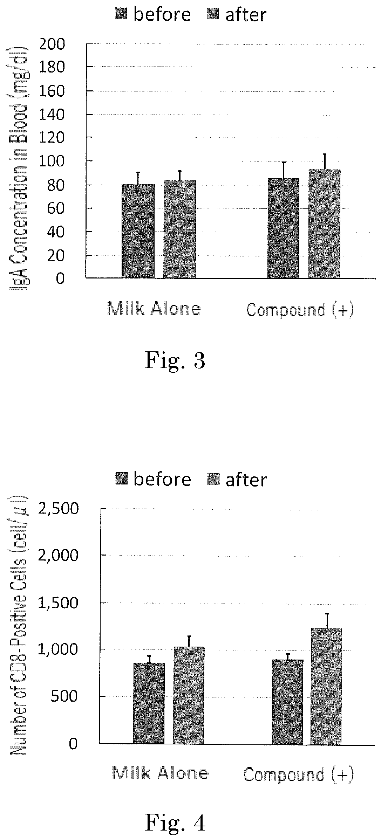

For IgA that is useful infection prevention against bacteria and viruses, has a low antigen specificity, and independently acts on mucous membrane of the whole body, to confirm the change of the IgA concentration in blood by ingesting the food or drink for infant containing nicotinamide mononucleotide, a test was conducted by the similar method under the similar condition to Example 1, and the IgA concentration in blood (mg/dl) was measured for each group and the average value was obtained. FIG. 3 illustrates the result. As illustrated in FIG. 3, in the group fed with the powdered milk to which nicotinamide mononucleotide was added, the IgA concentration more increased from before feeding with the milk to after feeding with the milk than in the group fed with the powdered milk alone. From this result, it was found that the food or drink for infant of the present invention was effective for increasing the IgA concentration in blood.

### Example 3. Evaluation of CD8-Positive T Cell Production promoting effect

While it has been known that the CD8-positive T cell plays a central role for removing pathogenic microorganisms, especially viruses, to confirm the change of the number of CD8-positive T cells in blood by ingesting the food or drink for infant containing nicotinamide mononucleotide, a test was conducted by the similar method under the similar condition to Example 1, and the number of CD8-positive T cells in blood (cell/µl) was measured for each group and the average value was obtained (excluding a subject whose number of CD8-positive T cells in blood before feeding with the powdered milk was out of a reference range corresponding to the age of the subject). FIG. 4 illustrates the result. As illustrated in FIG. 4, in the group fed with the powdered milk to which nicotinamide mononucleotide was added, the number of CD8-positive T cells more increased from before feeding with the milk to after feeding with the milk than in the group fed with the powdered milk alone. From this result, it was found that the food or drink for infant of the present invention was effective for introducing the CD8-positive T cells to increase the number of CD8-positive T cells in blood.

## Claims

1. A food or drink for infant that contains an effective dose of nicotinamide mononucleotide.

2. The food or drink for infant according to claim 1, used for improving an intestinal environment.

3. The food or drink for infant according to claim 1 or 2, used for enhancing an immune strength.

4. The food or drink for infant according to any one of claims 1 to 3, wherein
the infant is a baby.

5. The food or drink for infant according to any one of claims 1 to 4, wherein
the nicotinamide mononucleotide is contained with an amount provided in a range of 0.2 to 220 mg per day for one infant in terms of nicotinamide mononucleotide amount.

6. The food or drink for infant according to any one of claims 1 to 5, wherein
the food or drink for infant is a powdered milk for childcare.

7. A method for improving an intestinal environment of an infant, comprising
a step of causing an infant whose intestinal environment is deteriorated to ingest the food or drink for infant according to any one of claims 1 to 6.

8. A method for enhancing an immune strength of an infant, comprising
a step of causing an infant whose immune strength is low to ingest the food or drink for infant according to any one of claims 1 to 6.

9. The method according to claim 7 or 8, wherein
an intake of the food or drink for infant is an amount in a range of 0.2 to 220 mg per day for one infant in terms of nicotinamide mononucleotide amount contained in the food or drink.
